Europäisches Patentamt

European Patent Office

Office européen des brevets

(11) Publication number: **0 208 410**
**B1**

(12) **EUROPEAN PATENT SPECIFICATION**

(45) Date of publication of patent specification: **12.09.90**

(21) Application number: **86304245.3**

(22) Date of filing: **04.06.86**

(51) Int. Cl.⁵: **C 07 C 5/32,** C 07 C 15/46, C 07 C 5/333

(54) Hydrocarbon dehydrogenation process with oxidative reheat.

(30) Priority: **05.06.85 US 741332**
**02.05.86 US 858990**

(43) Date of publication of application:
**14.01.87 Bulletin 87/03**

(45) Publication of the grant of the patent:
**12.09.90 Bulletin 90/37**

(84) Designated Contracting States:
**AT BE CH DE FR GB IT LI NL SE**

(56) References cited:
**EP-A-0 144 476**
**US-A-1 869 681**
**US-A-3 855 330**
**US-A-4 376 225**
**US-A-4 435 607**
**US-A-4 565 898**

(73) Proprietor: **UOP**
**25 East Algonquin Road**
**Des Plaines Illinois 60017-5017 (US)**

(72) Inventor: **Ward, Dennis John**
**22 Windemere Lane**
**South Barrington Illinois 60010 (US)**

(74) Representative: **Brock, Peter William et al**
**URQUHART-DYKES & LORD 91 Wimpole Street**
**London W1M 8AH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Description

The invention relates to a hydrocarbon conversion process. In particular, the invention relates to a process for the catalytic dehydrogenation of an alkylaromatic hydrocarbon. The preferred use of the subject process is the dehydrogenation of ethylbenzene to styrene. The invention is specifically related to the manner in which the reactants are reheated to a desired temperature between beds of catalyst used in series in the endothermic process. The invention also relates to the use of selective hydrogen oxidation catalyst to heat reactants by the combustion of hydrogen.

The dehydrogenation of hydrocarbons is will described in the prior art, with both acrylic and aromatic hydrocarbons being thereby converted into the corresponding less saturated products. For instance dehydrogenation is performed commercially for the production of styrene from ethylbenzene to fulfill the sizable demand for this polymer precursor. The product styrene may be polymerized with itself or it may be copolymerized with butadiene, isoprene, acrylonitrile, etc. Processes for the dehydrogenation of alkylaromatic hydrocarbons are often integrated with an alkylation process which produces the alkylaromatic hydrocarbons.

US—A—3515766 and US—A—3409689 are pertinent for their showing of typical prior art catalytic steam dehydrogenation processes for alkylaromatics including ethylbenzene. These references describe the admixture of superheated steam into the feed hydrocarbon and the admixture of additional amounts of superheated steam with the reactants between sequential beds of dehydrogenation catalyst. These references also show an overall process flow into which the subject process could be integrated. Different dehydrogenation catalysts may be used in different beds as described in US—A—3223743.

It is also known in the prior art to pass oxygen into a dehydrogenation zone for the purpose of reacting the oxygen with hydrogen released during the dehydrogenation reaction to thereby liberate heat and to comsume hydrogen. The processes known to employ this technique utilize a hydrogen oxidation catalyst in an attempt to selectively oxidize the hydrogen rather than feed or product hydrocarbons also present in the dehydrogenation zone. For instance, US—A—3437703 discloses a dehydrogenation process which may utilize either a "homogeneous catalyst system" in which oxidation and dehydrogenation catalysts are admixed or a layered system of individual catalyst beds referred to as a "multi-bed" system. Similarly, US—A—3855330 discloses a dehydrogenation process using alternating bed of dehydrogenation catalyst and oxidation catalyst contained adjacent to one another in a single reactor. It is preferred that oxygen is introduced only after substantial conversion of the feed hydrocarbon, it is taught that it is desirable that oxygen does not come into contact with the dehydrogenation catalysts, and that the major part or all of the added oxygen is consumed within the bed of oxidation catalyst.

Another document disclosing a process carried out with alternating beds of dehydrogenation catalyst and selective hydrogen oxidation catalyst is US—A—4435607 which describes suitable catalysts in considerable detail. Both this document and US—A—3855330 are concerned chiefly with the existence of the alternating beds containing catalysts having different functions.

US—A—3502737 presents a process for the dehydrogenation of ethylbenzene which indicates that catalyst activity and stability are maintained by the careful control of the amount of oxygen which is present and by a reduction in the steam which is used in the reaction zone. An oxygen-containing gas such as air is supplied initially and at interstage points in a carefully controlled manner. It is believed that the teaching of this reference is limited to the use of a catalyst system comprising a physical admixture of the hydrogen oxidation catalyst and the dehydrogenation catalyst, with the presence of oxygen being credited with assisting in the prevention of carbon deposits on the surface of catalytically active sites of the dehydrogenation catalyst.

US—A—4376225 describes a catalytic dehydrogenation process in which selective hydrogen combustion is employed as a means of partial interstage reheating. The selective combustion is employed subsequent to conventional indirect heat exchange. It is performed close to the inlet of the dehydrogenation catalyst bed in order to minimize the time at which the reactants are held at a high temperature and to thus minimize thermal degradation reactions.

US—A—3904703 describes a dehydrogenation process comprising alternating layers of dehydrogenation catalyst and oxidation catalysts. This reference teaches performance is improved by inserting a layer of absorbent after each oxidation layer to remove water produced in the oxidation layer. This absorbent is regenerated during the periodic catalyst regenerations. No air is added to the process as an oxygen source as the oxygen released during dehydrogenation by the periodically regenerated catalyst is employed as the oxygen source.

The subject invention provides a method for revamping conventional dehydrogenation processes, which previously employed indirected heat exchange or steam addition to provide the necessary interstage heating, into an improved oxidation reheat mode of interstage reheating. The subject method will provide both yield and conversion compared to the prior art indirect heat exchange method of interstage heating. The subject invention may also be employed with beneficial effects in new process units to obtain improved catalytic action by the selective utilization of different dehydrogenation catalysts.

The process according to the invention is conducted in at least two reactors separated by conduits, by

(a) passing the feed hydrocarbon through a first reactor containing the first bed of dehydrogenation catalyst, to produce a first reactor effluent stream comprising the feed hydrocarbon, unsaturated product hydrocarbon and hydrogen;

(b) transporting the first reactor effluent stream through a first conduit into a second reactor, and contacting it with oxygen and the bed of selective hydrogen oxidation catalyst, to heat the effluent stream by oxidation of the hydrogen, passing the thereby heated first reactor effluent stream into contact with a second bed of dehydrogenation catalyst located adjacent to the bed of selective hydrogen oxidation catalyst, and producing a second reactor effluent stream comprising feed hydrocarbon, unsaturated product hydrocarbon and hydrogen;

(c) transporting the second reactor effluent stream through a second conduit into a third bed of dehydrogenation catalyst, which is larger than said second bed of dehydrogenation catalyst, and producing a third effluent stream comprising the feed hydrocarbon, unsaturated product hydrocarbon and hydrogen; and

(d) recovering the unsaturated product hydrocarbon.

By locating a bed of dehydrogenation catalyst adjacent to the bed of selective hydrogen oxidation catalyst employed in the reheat step, the reactants are brought into contact with the dehydrogenation catalyst and cooled immediately upon leaving the bed of oxidation catalyst. Only after this step are the reactants transported through the second conduit into the main bed of dehydrogenation catalyst in the third reactor. Impurity generating thermally promoted reactions are therefore minimized by reducing both the residence time prior to initial cooling and the temperature to which the reactants are exposed prior to the main dehydrogenation step.

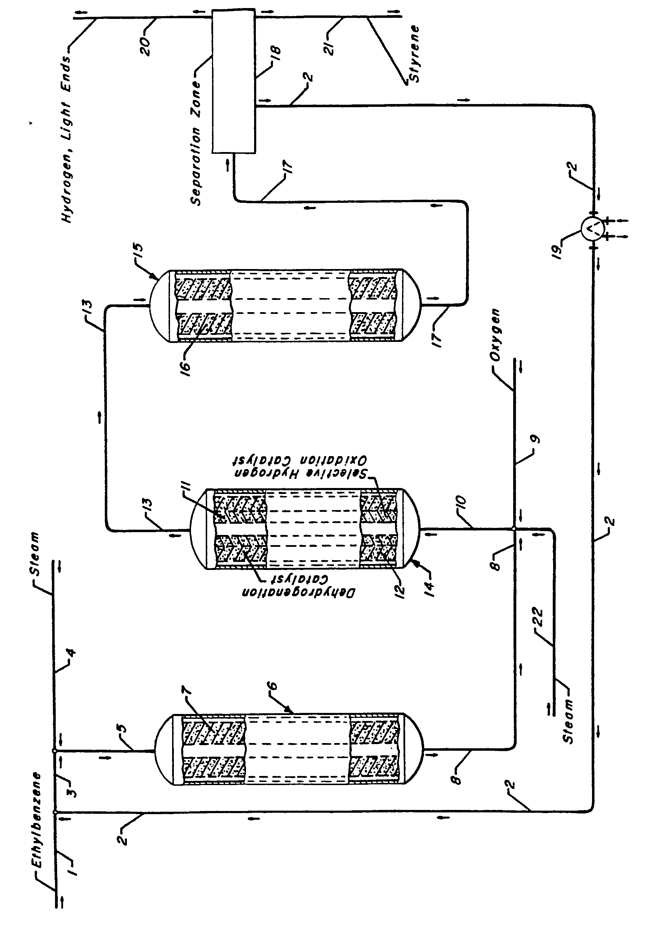

The drawing is a simplified process flow diagram illustrating the passage of ethylbenzene through a first reactor 6, with the endothermic dehydrogenation reaction producing a relatively low temperature stream which is passed into the reactor 14 for reheating by selective hydrogen oxidation. The heated stream then immediately flows into contact with dehydrogenation catalyst 12 present within this reheating reactor. After this initial limited dehydrogenation step, the reactants flow through line 13 into the third reactor 15 at a reduced temperature still sufficient for the desired remaining dehydrogenation in the thermal reactor.

Processes for the dehydrogenation of hydrocarbons, especially aromatic hydrocarbons, are in a widespread commercial use. For instance, large quantities of styrene are produced by the dehydrogenation of ethylbenzene. The resultant styrene may be polymerized with itself or it may be copolymerized with butadiene, isoprene, acrylonitrile, etc. Other aromatic hydrocarbons which may be dehydrogenated in much the same manner include diethylbenzene, ethyl toluene, propylbenzene, and isopropylbenzene. Catalytic dehydrogenation can also be employed to convert acyclic hydrocarbons such as ethane, propane, butane and $C_7$—$C_{15}$ paraffins to the corresponding mono-olefin. However, since the great majority of the present commercial dehydrogenation processes are employed in the dehydrogenation of ethylbenzene, the following description of the subject invention will be presented primarily in terms of dehydrogenation of ethylbenzene. This is not intended to exclude from the scope of the subject invention those other feed hydrocarbons set out herein or those having different ring structures including bicyclic compounds.

The dehydrogenation reaction is highly endothermic. Therefore, passing the reactants through the catalyst bed results in a decrease in the reactant temperature. The endothermicity of the reaction is such that the temperature decrease removes the reactants from the desired temperature range. The reactants are actually cooled to such an extent that the desired reaction does not occur at a commercially feasible rate. The desired or commercially necessary per pass conversion therefore can not be achieved by simply passing the reactants into contact with a single bed of dehydrogenation catalyst. For this reason, it has become standard commercial practice to in some manner perform interstage reheating. In interstage reheating the reactant effluent of a first bed of catalyst is heated to the desired inlet temperature of a second downstream bed of catalyst. This reheating can be performed through direct heat exchange as by the admixture of high temperature steam into the reactant stream emerging from the first catalyst bed. This accomplishes the desired heating, but has a number of drawbacks including the utilities cost of producing the high temperature steam. It also increases the amount of steam which must be condensed to recover the product alkylaromatic hydrocarbons from the effluent stream and increases the total amount of material flowing through the reaction zone, thereby making it more difficult to maintain desired low pressures within the reaction zone.

Another method of interstage reheating comprises the use of indirect heat exchange. In this method the effluent from a dehydrogenation zone is passed through a heat exchanger in which it is heated, and the reactants are then passed into the subsequent dehydrogenation zone. The high temperature fluid employed in this indirect heat exchange method may be high temperature steam, combustion gases, a high temperature process stream or other readily available high temperature fluids. This method of interstage heating does not dilute the reactants but does impose some pressure drop in the system and can expose the reactants to undesirably high temperatures.

A third method of interstage heating is the oxidation reheat method. This is a newer method which it is believed has not been employed commercially. The driving force for employing the oxidation reheat method is the recognition that the combustion of the hydrogen generated in the dehydrogenation process performs two functions which are beneficial in the dehydrogenation process. First, the consumption of the hydrogen is beneficial in shifting the equilibrium of the dehydrogenation reaction to favor increased amounts of dehydrogenation. Second, the combustion of the hydrogen will release heat sufficient to reheat the reactants to the desired dehydrogenation conditions. The oxidation is preferably accomplished in the presence of a catalyst which selectively promotes the oxidation of hydrogen as compared to the destructive combustion or oxidation of the more valuable feed and product hydrocarbons. The selective combustion method of interstage reheating presents a more economical dehydrogenation process. It is therefore expected that oxidative reheat will to a significant extent supplant indirect heat exchange as a method of performing the required interstage heating. Therefore, a large number of existing alkylaromatic dehydrogenation process units will be converted from indirect heat exchange to oxidative reheat interstage heating. It is an objective of the subject invention to provide a method and a resulting process for this conversion or revamping of existing dehydrogenation process units. It is also an objective of the subject invention to provide a method for increasing the processing capacity of existing alkylaromatic dehydrogenation process units. It is another objective of the subject invention to reduce the amount of thermal degradation of reactants which occurs during interstage reheating.

These and other objectives of the subject invention are achieved through the use of a separate intermediate reactor which contains adjacent beds of selective oxidation and dehydrogenation catalysts. The amount of oxidation catalyst in this inter-heating reactor is sufficient to provide the necessary reheat of the reactant stream. The amount of dehydrogenation catalyst present in this inter-heating reactor is sufficient to provide, at the chosen operating conditions, only a limited portion of the dehydrogenation conversion which is desired in the next stage of the dehydrogenation process. That is, the amount of dehydrogenation catalyst present in the inter-heating reactor is only sufficient to result in enough of the dehydrogenation reaction occurring that the reactants are cooled to a temperature at which the undesirable thermal reactions occur at only a minimal rate but which is still high enough to affect a very significant amount of dehydrogenation in the subsequent bed of dehydrogenation catalyst.

The subject invention relates to a method which may be employed in revamping commercial ethylbenzene dehydrogenation units from indirect heat exchange methods of interstage heating to the combustive reheat method of interstage reheating. In such a revamping, the simplest approach would be the substitution of a bed of the selective oxidation catalyst for the indirect heat exchange means previously used. The subject invention builds upon and improves this simple substitution in two ways. After the simple substitution of the oxidative heating method for heat exchangers, the problem present in the prior art indirect heat exchange methods of overheating and long thermal residence time still remains. By providing a bed of dehydrogenation catalyst in close contact with a bed of selective oxidation catalyst, the subject invention quickly cools the reheated reactants sufficiently to avoid the problems of thermal cracking reactions. This increases the quality of the produce and increases the yield as shown below. Second, the dehydrogenation catalyst located adjacent to the selective hydrogen oxidation catalyst is in addition to the catalyst previously present in the subsequent dehydrogenation reactor. The total amount of dehydrogenation catalyst employed in the process is thereby increased. This allows the reaction to proceed somewhat further in each subsequent bed of catalyst and therefore the overall conversion in the process may be increased.

A third advantage offered by the subject invention is the ability to more closely tailor the selection of catalyst to the reaction conditions at which the dehydrogenation reaction is being performed. More specifically, the subject process flow allows a catalyst which is more tolerant of high temperature and/or which has a better high temperature selectivity pattern to be employed in the reheating reactor while a different catalyst which is more active and perhaps less selective can be employed in the subsequent dehydrogenation reactor. With specific reference to the drawings, this would mean that the catalyst present in bed 12 would be different from the catalyst present in bed 16. This ability to select preferred catalyst and align them with the operating conditions should also result in improved selectivity and conversion in commercial processes.

The cooling effect provided by the dehydrogenation catalyst should cool the effluent of the selective hydrogenation oxidation catalyst bed by at least 18 Fahrenheit degrees (10 Celsius degrees). It is preferred that the reactants are cooled between 27 and 72 Fahrenheit degrees (15 and 40 Celsius degrees) during passage through the bed of dehydrogenation catalyst employed within this interheating reactor and located adjacent to the oxidation catalyst. In this regard, it should be pointed out that it is preferred that the effluent of the previous dehydrogenation reactor is preferably heated to a temperature about 1148 Fahrenheit degrees (620 Celsius degrees) during passage through the bed of selective hydrogen oxidation catalyst. After these reactants have passed through the adjacent bed of dehydrogenation catalyst in which they are cooled, it is preferred that the reactants will now have a temperature below about 1130 Fahrenheit degrees (610 Celsius degrees) prior to passage through the conduit which links the reheating reactor to the subsequent dehydrogenation reactor. To provide a sufficiently high inlet temperature in the subsequent dehydrogenation reactor the effluent stream flowing through the conduit must have a temperature above about 1076 Fahrenheit degrees (580 Celsius degrees).

For example the feed stream to the first reactor may have a temperature of about 620°C. The effluent of this dehydrogenation reactor would have a temperature of about 540°C. This stream is then heated to about 630°C in the reheating or second reactor (including cooling within second reactor). In a third reactor used solely for dehydrogenation the reactants are cooled to approximately 590°C. The effluent of this third reactor is heated from 575°C to about 640°C in a fourth reactor (second reheating reactor) before being cooled from 640 to 610°C in the final dehydrogenation reactor.

The drawing presents a simplified process flow diagram of a preferred embodiment of the invention. In this embodiment, the feed stream of relatively high purity ethylbenzene is charged to the process in line 1 and is then admixed with a recycle hydrocarbon stream carried by line 2. The admixture of the feed stream and recycle stream may if desired be passed through an indirect heat exchange means not shown for the purpose of heating the reactants. The admixture of line 3 is then combined with a stream of high temperature steam carried by line 4, and the steam and ethylbenzene is passed into the first reactor 6 via line 5. In reactor 6, the entering steam and ethylbenzene flow into the vertical cylindrical center pipe volume of the radial flow reactor. The reactants then pass horizontally outward through the annular catalyst bed 7 of dehydrogenation catalyst. This contacting at dehydrogenation promoting conditions results in the conversion of some of the charged ethylbenzene to styrene and hydrogen. The products of the first contacting step and residual compounds are collected in an annular void volume which surrounds the outer catalyst retaining screen of the catalyst bed 7. The reactants then flow out of this annular reactant collection volume and emerge from the reactor 1 through line 8.

The cooling caused by the dehydrogenation reaction performed in the first reactor necessitates the heating of the reactants flowing through line 8 prior to their contacting with the downstream dehydrogenation catalyst. In the subject process, the reactants flowing through line 8 are admixed with a small amount of high temperature steam from line 22 and an oxygen-containing gas such as air flowing through line 9. The admixture of residual ethylbenzene, product styrene and hydrogen, steam and oxygen flows through line 10 into a second reactor 14 referred to herein as a reheating reactor. The charge admixture of the reactor 14 flows upward into the center pipe volume located along the vertical axis of the reactor. The reactants then flow radially outward through a catalyst retaining screen into a bed of selective hydrogen oxidation catalyst 11. The selective oxidation of the hydrogen which occurs within this catalyst bed results in the consumption of hydrogen and a significant heating of the total mass of material flowing through this catalyst bed. The ethylbenzene present in the reactant stream is therefore heated to at least the desired inlet temperature for subsequent dehydrogenation operations. The effluent of the bed of selective hydrogen oxidation catalyst passes through another catalyst retaining screen and immediately enters into a bed 12 of dehydrogenation catalyst. These two adjacent catalyst beds are preferably separated only by the catalyst retaining scrren which is located between them. The thus heated ethylbenzene therefore flows into bed 12 of dehydrogenation catalyst at a temperature sufficient to achieve the desired degree of dehydrogenation during contact with the dehydrogenation catalyst in this and a subsequent reactor. This contacting in the reactor 14 results in the production of an increased amount of styrene and hydrogen. The effluent of bed 12 emerges through a third catalyst retaining screen into the annular reactant collection volume surrounding the catalyst beds and then flows upward and emerges through line 13.

The effluent of the second reactor is passed directly into the third reactor 15 without intermediate heating by any means including steam injection. This is because the effluent of the second bed of dehydrogenation catalyst is at an acceptably high temperature to enter the third bed of dehydrogenation catalyst. The reactants flow into the reactor 15 wherein they pass radially through the bed 16 of dehydrogenation catalyst. Further conversion of ethylbenzene to styrene and hydrogen occurs within the third reactor 15. This produces a third effluent stream transported through line 17 to the separation zone 18. The separation zone preferably comprises equipment similar to that described in the previously cited references. In this equipment the compounds flowing through line 17 are first subjected to indirect heat exchange sufficient to cause condensation of a very large percentage of the water and $C_6$ plys hydrocarbons entering the separation zone through line 17. The water and hydrocarbons are separated by decantation, with the resultant hydrocarbon stream being passed into a multi-column fractionation zone. Ethylbenzene and/or cyclic compounds such as toluene may be recycled through line 2. The recycle stream of line 2 would preferably be heated and vaporized in the indirect heat exchange means 19 prior to admixture with the feed stream. The uncombusted hydrogen and light ends such as methane or ethane produced in the dehydrogenation reactors are withdrawn from the separation zone through line 20. The product styrene is removed through line 21.

A preferred embodiment of the subject invention may accordingly be described as a process for the dehydrogenation of ethylbenzene which comprises the steps of passing a feed stream comprising ethylbenzene and steam through a first reactor in which the feed stream is passed through a first bed of dehydrogenation catalyst maintained at dehydrogenation conditions and thereby producing a first reactor effluent stream comprising ethylbenzene, steam, styrene, and hydrogen; transporting the first reactor effluent stream through a first conduit, which is external to the first reactor, into a second reactor, admixing an oxygen-containing gas into the first reactor effluent stream and then contacting the first reactor effluent stream with a bed of selective hydrogen oxidation catalyst at hydrogen oxidation promoting conditions which effect a heating of the first reactor effluent stream to a temperature above 605 degrees Celsius, passing the effluent of the selective hydrogen oxidation catalyst bed into contact with a second bed of dehydrogenation catalyst located adjacent to the bed of selective hydrogen oxidation catalyst, and producing a second reactor effluent stream having a temperature at least 10 Celsius degrees lower than the effluent of the adjacent selective hydrogen oxidation catalyst bed and comprising ethlybenzene, steam, styrene and hydrogen; passing the second reactor effluent stream into a third reactor through a second conduit, which is external to the second and third reactors, and contacting the second reactor effluent stream with a third bed of dehydrogenation catalyst maintained at dehydrogenation conditions and producing a third reactor effluent stream comprising ethylbenzene, styrene and hydrogen; and recovering the product styrene. As an illustration the effluent of the bed of selective oxidation catalyst may have a temperature of 650 degrees Celsius and be cooled to 620 degrees Celsius in the adjacent second bed of dehydrogenation catalyst. A temperature reduction of 10 Celsius degrees will normally cut the rate of thermal degradation by at least 50 percent.

Example 1 and Comparative Example 1

The following example compares the operations which result from revamping an existing multivessel process for the dehydrogenation of ethylbenzene. The preexisting unit would be similar to that shown in the drawing except that the vessel 14 would not exist since a fired heater or other heating means would be employed to heat the effluent of the first dehydrogenation reactor 6. Such interheaters are shown in previously cited US—A—4376225. As it is impractical to build commercial scale units just to test new concepts the following comparisons are based upon engineering calculations premised on results gathered during the design and operation of commercial scale ethylbenzene dehydrogenation units, pilot plant studies using oxidative reheating methods and results published in the scientific literature. They are believed to fairly and accurately portray the relative performance of the two systems.

In the prior art method of employing oxidative reheat the interheater is replaced by a reactor 14 (using the drawing as a guide) containing only a bed 11 of selective oxidation catalyst designed and operated in a manner which results in the heating of the effluent of the reactor 6 to the desired inlet temperature (645 degrees Celsius) of the reactor 15. In the improved system of the subject invention the reactor 14 also contains the bed 12 of dehydrogenation catalyst. In both cases the feed to the reactor 14 contains 5 mole percent ethylbenzene, 3.5 mole percent styrene, 3.5 mole percent hydrogen and 88 mole percent water and has a pressure of 0.7 atm a. (70.9 kPa).

In the prior art system the effluent of reactor 14 is fed to the dehydrogenation catalyst bed of reactor 15 at an inlet temperature of 645 degrees Celsius. The residence time of the gas at this temperature prior to contacting the dehydrogenation catalyst is about 15 seconds. In the process according to the invention the catalyst contacts the bed 12 of dehydrogenation catalyst less than 0.05 seconds after leaving the oxidation catalyst bed 11, and the gas stream of line 13 contacts the dehydrogenation catalyst of reactor 15 at an inlet temperature of 630 degrees Celsius. Due to the increased high temperature residence time which occurs within line 13 and vessels 14 and 15 prior to cooling the effluent of the oxidation catalyst bed the prior art process will produce more benzene, toluene and xylenes. The rate of benzene and toluene production will be 46 percent greater than in the invention. This increased byproduct production results in a definite yield advantage for the subject process. In addition the rate of o-xylene production will be reduced in less than half of that in the prior art system. As o-xylene is very difficult to separate from styrene by fractional distillation any o-xylene production results in a corresponding decrease in styrene product purity. The subject process therefore increases both product quality and product yield.

This comparative example also illustrates the sizable residence time which results from operation at a low pressure. Low pressure operations require large conduits and moderate gas velocities to prevent sizable pressure drops through the overall process. The non-selective thermal reactions continue during this lengthy residence time. In considering the effectiveness of the subject method it must be recognized that in the temperature range under consideration a reduction in the temperature of the reactants of from 10 to 20 Celsius degrees is sufficient to reduce the rates of thermal degradation to one quarter or one-sixth of the rate at the higher temperature. When this is combined with at least a 100-fold decrease in the residence time the result is a tremendous decrease in the total amount of byproducts produced in thermal reactions intermediate the two catalyst beds.

Comparative Example 2

A Styrene unit having two reactors, each containing 46 cubic meters of commercially available dehydrogenation catalyst, processes 47 metric tons per hour (MTH) of charge stock containing 0.4 MTH of toluene and 0.5 MTH of styrene, together with 56 tons per hour of steam. The inlet temperature to each reactor is 645°C and the outlet pressure of the last reactor is about one-half atmosphere absolute (50.7 kPa). The inlet temperature of the second dehydrogenation bed is achieved by admixing approximately 11 MTH of air with the effluent of the first reactor and passing the admixture over a radial flow bed of special oxidation catalyst formulated to selectively react the oxygen from the air with hydrogen from the first dehydrogenation reactor. The result of this operation is the reaction (or conversion) of 33 MTH of ethylbenzene (70 percent) into 30.5 MTH of styrene. The operation is expected to continue for one year with gradually increasing temperature being required at the inlet of the second dehydrogenation reactor due to the formulation of irreversible "coke" on that catalyst bed due to reactive thermal reaction products occurring in the transfer line between the oxidation catalyst and the second dehydrogenation reactor. In addition, there is a conversion of approximately 0.05 MTH of ethylbenzene to benzene, toluene and other by-products in the transfer line at the conditions indicated above which increases to 0.06 MTH at the end of run conditions.

Example 2

The operations are the same as Comparative Example 2 above except that an 18 cubic meter radial flow bed of dehydrogenation catalyst is included directly adjacent to and after the bed of special oxidation catalyst. While processing the same charge stock at the same first reactor conditions, and again adding 11 MTH of air, the transfer temperature is now 626°C (the oxidation catalyst outlet temperature is still 645°C) and the results after the second 46 cubic meter bed of catalyst is the reaction of 35.6 metric tons of ethylbenzene producing 32.4 MTH of styrene, approximately eight percent more styrene than in Comparative Example 2, with the same steam air and hydrocarbon charge. In addition, the dehydrogenation catalyst is expected to last two years before changeout is required due to the relatively low coke precursor generation in the transfer line (less than two-thirds of that of Comparative Example 2). The loss of ethylbenzene to other products in the transfer line to by-products is about 0.03 MTH at the conditions indicated above.

Comparative Example 3

The same conditions and catalysts as in Example 2 above are employed except the air rate is increased to 13.5 MTH and essentially all of the hydrogen is reacted and no higher temperature can be easily produced. The transfer pipe temperature at this condition is about 660°C. The ethylbenzene conversion rate is 34.6 MTH producing 32.1 MTH of styrene. The dehydrogenation catalyst is expected to last less than six months before changing is required and the loss of ethylbenzene to by-products in the transfer line of 0.07 MTH at these conditions.

In summary, the above examples illustrate the subject invention provides a higher purity product, less by-product production, longer catalyst life and the ability to significantly increase the capacity of the existing process units.

The total amount of dehydrogenation catalyst employed in the process may be divided into ten or more separate beds, but the dehydrogenation zone preferably comprises three catalyst beds with means for the intermediate addition and admixture of any added steam and the oxygen supply streams. Suitable systems for this may be patterned after those presented in US—A—3498775, US—A—3515763 and US—A—3751232. The catalyst beds may be contained in separate reaction vessels or they may be enclosed within a larger overall vessel or structure. The use of radial flow catalyst beds in a stacked configuration in a single overall vessel is preferred. In any event, the effluent stream of each reactor flows through a conduit which is external to the immediately upstream and downstream reactors. The conduits are therefore at least partially located outside of the reactor vessel which surrounds the catalyst beds.

The term "conduit" is used herein to refer to conventional cylindrical piping and is not intended to include internal structures or passageways within a process vessel suitable for process flow. The exact structure would depend on the best method of revamping the existing commercial units or of operating new units. As shown above the residence time of the reactants in a conduit can be extensive. The residence times can range from over 0.5 second to 20 seconds or more. For proper utilization of the subject invention, it is preferred that the residence time within the conduit of the effluent of the bed of dehydrogenation catalyst which is adjacent to the bed of oxidation catalyst is at least 1.0 seconds. This is the time in the second conduit prior to contacting the third bed of dehydrogenation catalyst.

Dehydrogenation catalysts for use with alkylaromatic hydrocarbons generally consist of one or more metallic components selected from Groups VI and VIII of the Periodic Table. One typical catalyst for the dehydrogenation of alkylaromatics comprises 85% by weight ferric oxide, 2% chromia, 12% potassium hydroxide and 1% sodium hydroxide. A second dehydrogenation catalyst, which is used commercially, consists of 87—90% ferric oxide, 2—3% chromium oxide and from 8—10% potassium oxide. A third typical catalyst comprises 90% by weight iron oxide, 4% chromia and 6% potassium carbonate. Methods for preparing suitable catalysts are well known in the art. This is demonstrated by the teachings of US—A—3387053 which describes the manufacture of a catalytic composite of at least 35 wt.% iron oxide as an active catalytic agent, from about 1—8 wt.% zinc or copper oxide, about 0.5—50 wt.% of an alkali promoter, and from about 1—5 wt.% chromic oxide as a stabilizer and a binding agent. US—A—4467046 also describes a catalyst for the dehydrogenation of ethylbenzene in the presence of steam. This catalyst contains 15 to 30 wt.% potassium oxide, 2 to 8% cerium oxide, 1.5 to 6% molybdenum oxide, 1 to 4% calcium carbonate with the balance iron oxide.

Dehydrogenation conditions for alkylaromatic hydrocarbons in general include a temperature of 538° to 750°C (1000°—1382°F) and preferably 565° to 675°C (1050°F). The temperature required for efficient operation of any specific dehydrogenation process will depend on the feed hydrocarbon and the activity of the catalyst employed. The pressure maintained within the dehydrogenation zone may range from 100 to 750 mm Hg (13.3 to 100 kPa), with a preferred range of pressures being from 250 to 700 mm Hg (33.3 to 93.3 kPa). The operating pressure within the dehydrogenation zone is measured at the inlet, midsection, and outlet of the zone to thereby provide an approximately average pressure. The combined feed stream is charged to the dehydrogenation zone at a liquid hourly spaced velocity, based on liquid hydrocarbon charge at 60°F (15.6°C), of 0.1 to 2.0 $hr^{-1}$, and preferably from 0.2 to 1.0 $hr^{-1}$.

An alkylaromatic hydrocarbon to be dehydrogenated is preferably admixed with superheated steam to counteract the temperature lowering effect of the endothermic dehydrogenation reaction. The presence of steam has also been described as benefiting the stability of the dehydrogenation catalyst by preventing the accumulation of carbon deposits. Preferably, the steam is admixed with the other components of the feed stream at a rate of 0.5 to 1.7 kg of steam per kg of feed hydrocarbon. Other quantities of steam may be added after one or more subsequent beds if desired. However, the dehydrogenation zone effluent stream should contain less than about 3 kg of steam per kg of product hydrocarbon and preferably less than 2 kg of steam per kg of product hydrocarbon.

The effluent stream removed from the last dehydrogenation zone is normally heat exchanged for the purpose of lowering its temperature for the recovery of heat. The effluent stream may be heat exchanged against a stream of steam, a reactant stream of this or another process or used as a heat source for fractionation, etc. Commercially, the effluent stream is often passed through several heat exchangers thereby heating a number of different streams. This heat exchange is performed subject to the constraints set out above. The heat exchange performed downstream of the first compression means should cool the dehydrogenation zone effluent stream sufficiently to affect the condensation of at least 95 mole percent of the feed and product $C_6$-plus hydrocarbons and also at least 95 mole percent of the water vapor. The use of a quench zone to accomplish this condensation is not preferred. Essentially all of the styrene or other product hydrocarbon, most water and other readily condensible compounds present in the effluent stream are thereby converted to liquids. This produces a mixed phase stream which is passed into a phase separation vessel. This procedure allows the facile crude separation by decantation of the hydrocarbons from the water and hydrogen present in the effluent stream. The sytrene present in the dehydrogenation zone effluent stream becomes part of a hydrocarbon stream which is withdrawn from the separation vessel and transferred to the proper separation facilities. Preferably, the styrene is recovered from the hydrocarbon stream by using one of the several fractionation sytems known in the art. This fractionation will preferably yield a relatively pure stream of ethylbenzene, which is recycled, and an additional stream comprising benzene and toluene. These two aromatic hydrocarbons are by-products of the dehydrogenation reaction. They may be recycled in part as taught in US—A—3409689 and GB—A—1238602 or entirely rejected from the process. Styrene is recovered as a third stream, which is withdrawn from the process. If desired, methods other than fractionation may be used to recover the styrene. For instance US—A—3784620 teaches the separation of styrene and ethylbenzene through the use of a polyamide permeation membrane such as nylon-6 and nylon 6,10. US—A—3513213 teaches a separatory method employing a liquid-liquid extraction in which anhydrous silver fluoroborate is used as the solvent. Similar separatory methods utilizing cuprous flurorborates and cuprous fluorophosphates are described in US—A—3517079, —3517080 and —3517081.

The recovery of styrene through the use of fractionation is described in several references including US—A—3525776. In this reference, the hydrocarbonaceous phase removed from the phase separation zone is passed into a first column referred to as a benzene-toluene column. This column is operated at a subatmospheric pressure to allow its operation at lower temperatures and hence reduce the rate of styrene polymerization. Various inhibitors such as elemental sulfur, 2,4-dinitrophenol or a mixture of N-nitroso diphenylamine and a dinitroso-o-cresol are injected into the column for this same purpose. Sulfur can also be introduced into this column by returning at least a portion of the high molecular weight material separated from the bottoms stream of a styrene purification column. A more detailed description of this is contained in US—A—3476656; —3408263; and —3398063. There is effected within the benzene-toluene column a separation of benzene and toluene from the effluent to produce an overhead stream which is substantially free of styrene and ethylbenzene. This stream preferably contains at least 95 mole percent benzene and toluene. The bottoms of the benzene-toluene column is passed into a second fractionation column from which ethylbenzene is removed as an overhead product and recycled. The bottoms stream of this column is then purified to obtain the styrene. Product recovery techniques directed to the recovery of vinyltoluene via fractionation and the use of chemical additives to inhibit polymerization are described in US—A—4417085 and —4492675. The use of inhibitors and alternative fractionation techniques for readily polymerizable vinyl aromatic compounds is also described in US—A—4469558.

For the dehydrogenation of normal paraffins, the reaction zone preferably comprises at least one radial flow reactor in which the catalyst gradually moves downward by gravity flow to allow the continuous replacement of used dehydrogenation catalyst with catalyst having a higher activity. It is preferred that the reactants make at least two passes through dehydrogenation catalyst beds within the reaction zone. A detailed description of moving bed reactors of this type may be obtained by reference to US—A—3647680; —3706536; —3825116; —3839196; —3839197; —3854887; —3856662; and —3978150.

The particular dehydrogenation conditions employed within the reaction zone may vary depending on such factors as the catalyst activity, feed carbon number, and the desired conversion. In the dehydrogenation of normal paraffins it is preferred to maintain a hydrogen to hydrocarbon mole ratio at the inlet to the reaction zone of from 0.5 to 6.0:1.0. It is preferred to not add any steam with the feed stream. The reaction zone conditions normally employed for dehydrogenation of propane and butane and other paraffins include a temperature of from 400 to 700 degrees Celsius, a pressure of from 0.5 to 10 atmospheres absolute (50.6 to 1013 kPa) and a liquid hourly spaced velocity of 1 to 20. The preferred operating temperature for propane and butane is within the range of from about 550 to 660 degrees Celsius, and the preferred operating pressure is 0.5 to 2 atmospheres absolute (50.5 to 202.6 kPa).

The preferred paraffin dehydrogenation catalyst is comprised of a platinum group component, preferably platinum, a tin component and an alkali metal component with a porous inorganic carrier material. Other catalytic compositions may be used within this zone if desired. The preferred catalyst contains an alkali metal component chosen from cesium, rubidium, potassium, sodium and lithium. The preferred alkali metal is normally chosen from lithium and potassium, with potassium being preferred for isobutane. Preferred dehydrogenation catalysts comprise an alkali metal and a halogen such as potassium and chlorine in addition to the tin and platinum group components. The perparation and use of dehydrogenation catalysts is well known to those skilled in the art and further details as to suitable catalyst compositions and operating conditions are available in standard references (US—A—4430517; —4486547; —4469811; and —4438288). The product olefins may be recovered by fractional distillation or by selective reaction with another compound in accordance with known methods.

The oxygen supply stream may be air but is preferably a gas having a higher oxygen content than air. It is preferred that the oxygen supply stream has a nitrogen content less than 10 mole percent, with the use of substantially pure oxygen being preferred if it is economically viable. The preferred oxygen concentration in the oxygen supply stream is primarily a matter of economics and would be determined by a comparison of the advantage of having pure oxygen to the cost of obtaining the oxygen. The basic disadvantages of the presence of nitrogen are the dilution of the hydrogen-containing gas stream removed from the product separation vessel and the fact that the nitrogen passes through the dehydrogenation zone thereby increasing the pressure drop through the catalyst bed and the absolute pressure being maintained within the dehydrogenation zone. On the other hand, the presence of nitrogen favorably affects the equilibrium conversion level by acting as a diluent.

The oxidation catalyst employed in the subject process to promote the interstage hydrogen oxidation may be any commercially suitable catalyst which meets the required standards for stability and activity and which possesses high selectivity for the oxidation of hydrogen as compared with the oxidation of the feed or product hydrocarbon. That is, the oxidation catalyst must have a high selectivity for the oxidation of hydrogen with only small amounts of the feed of product hydrocarbon being oxidized. The oxidation catalyst will have a different composition than the dehydrogenation catalyst. The preferred oxidation catalyst comprises a Group VIII noble metal and a metal or metal cation which possesses a crystal ionic radius greater thant 1.35 angstroms, with both of these materials being present in small amounts on a refractory solid support. The preferred Group VIII metals are platinum and palladium, but the use of ruthenium, rhodium, osmium and iridium is also contemplated. The Group VIII metal is preferably present in an amount equal to 0.01 to 5.0 wt.% of the finished catalyst. The metal or metal cation having a radius greater than 1.35 angstroms is preferably chosen from Group IA or IIA and is present in an amount equal to 0.01 to 20 wt.% of the finished catalyst. This component of the catalyst is preferably barium, but the use of other metals including rubidium or cesium is also contemplated.

The preferred solid support is alumina having a surface area between 1 and 300 m²/g, an apparent bulk density of between 0.2 and 1.5 g/cc, and an average pore size greater than 20 angstroms. The metal-containing components are preferably impregnated into solid particles of the solid support by immersion in an aqueous solution followed by drying and calcination at a temperature of from 500° to 600°C in air. The support may be in the form of spheres, pellets or extrudates. The total amount of oxidation catalyst present within the dehydrogenation zone is preferably less than 30 wt.% of the total amount of dehydrogenation catalyst and more preferably is between 5 and 15 wt.% of this total amount of dehydrogenation catalyst. Further information on the composition of a suitable selective oxidation catalyst is provided in US—A—4435607 and —4565898, which are incorporated herein by reference.

The conditions utilized during the contacting of the reactant streams with the oxidation catalyst will be set to a large extend by the previously referred to dehydrogenation conditions. The preferred outlet temperature of any bed of oxidation catalyst is the preferred inlet of the immediately downstream bed of dehydrogenation catalyst. The temperature rise across any bed of oxidation catalyst is preferably less than 100 Celsius degrees. The liquid hourly space velocity, based on the liquid hydrocarbon charge at 60°F (16°C), is preferably between 2 and 10 hr$^{-1}$. It is preferred that substantially all of the oxygen which enters a bed of oxidation catalyst is consumed within that bed of oxidation catalyst and that the effluent stream of any bed of oxidation catalyst contains less than 0.1 mole percent oxygen. The total moles of oxygen charged to the dehydrogenation zone is preferably less than 50% of the total moles of hydrogen available within the dehydrogenation zone for combustion and is therefore dependent on the converion achieved in the dehydrogenation zone and the amount of hydrogen lost in solution or in any off-gas streams. This available hydrogen is the sum of any hydrogen recycled to the dehydrogenation zone and the hydrogen produced in all but the last bed of dehydrogenation catalyst. Preferably the oxygen charged to the dehydrogenation zone is equal to about 20 to 50 mole percent of the thus-defined available hydrogen. As used herein, the term "substantially all" is intended to indicate a major fraction of the indicated chemical compound(s) which have been acted upon in the manner described, with this major fraction preferably being over 90 mole percent and more preferably over 95 mole percent. As previously mentioned, the subject process is not limited to the production of styrene and may be used to produce paramethylstyrene by dehydrogenation of ethyltoluene or for the production of other unsaturated product hydrocarbons.

## Claims

1. A process for the dehydrogenation of a feed hydrocarbon by passing said hydrocarbon in sequence through a first bed of dehydrogenation catalyst to form a mixture of feed hydrocarbon, an unsaturated product hydrocarbon, and hydrogen; a bed of selective hydrogen oxidation catalyst in the precence of oxygen to oxidise said hydrogen; and a further bed of dehydrogenation catalyst to form further unsaturated product hydrocarbon; characterized in that said process is conducted in at least two reactors separated by conduits, by

(a) passing the feed hydrocarbon into a first reactor containing the first bed of dehydrogenation catalyst, to produce a first reactor effluent stream comprising the feed hydrocarbon, unsaturated product hydrocarbon and hydrogen;

(b) transporting the first reactor effluent stream through a first conduit into a second reactor, and contacting it with oxygen and the bed of selective hydrogen oxidation catalyst, to heat the effluent stream by oxidation of the hydrogen, passing the thereby heated first effluent stream into contact with a second bed of dehydrogenation catalyst located adjacent to the bed of selective hydrogen oxidation catalyst, and producing a second reactor effluent stream comprising feed hydrocarbon, unsaturated product hydrocarbon and hydrogen;

(c) transporting the second reactor effluent stream through a second conduit into a third bed of dehydrogenation catalyst, which is larger than said second bed of dehydrogenation catalyst, and producing a third reactor effluent stream comprising the feed hydrocarbon, unsaturated product hydrocarbon and hydrogen; and,

(d) recovering the unsaturated product hydrocarbon.

2. A process according to claim 1 characterized in that oxygen and steam are mixed with the first reactor effluent stream within said first conduit.

3. A process according to claim 1 or 2 characterized in that the feed hydrocarbon is an alkylaromatic hydrocarbon.

4. A process according to any one of claims 1 to 3 characterized in that said bed of selective hydrogen oxidation catalyst and the second bed of dehydrogenation catalyst are adjacent annular beds of catalyst having a porous catalyst retaining screen in common.

5. A process according to claim 1 characterized in that the feed hydrocarbon is ethylbenzene, whereby the first reactor effluent stream comprises ethylbenzene, steam, styrene and hydrogen, the bed of selective hydrogen oxidation catalyst is operated to heat the first reactor effluent stream to a temperature above 605 degrees Celsius, and the effluent of the selective hydrogen oxidation catalyst bed is passed into contact with the second bed of dehydrogenation catalyst whereby the second reactor effluent stream has a temperature at least 10 Celsius degrees lower than the effluent of the selective hydrogen oxidation catalyst bed.

## Patentansprüche

1. Verfahren zur Dehydrierung eines Beschickungskohlenwasserstoffes durch Führen des Kohlenwasserstoffes in Aufeinanderfolge durch ein erstes Bett eines Dehydrierungskatalysators zwecks Bildung eines Gemisches aus Beschickungskohlenwasserstoff, einem ungesättigten Produktkohlenwasserstoff und Wasserstoff: ein Bett eines selektiven Wasserstoffoxydationskatalysators in Gegenwart von Sauerstoff zwecks Oxydation des Wasserstoffes; und ein weiteres Bett eines Dehydrierungskatalysators zwecks Bildung eines weiteren ungesättigten Produktkohlenwasserstoffes, dadurch gekennzeichnet, daß das Verfahren in wenigstens zwei, durch Lietungen getrennten Reaktoren durchgeführt wird, indem man

(a) den Beschickungskohlenwasserstoff in einen ersten, das erste Dehydrierungskatalysatorbett enthaltenden Reaktor zwecks Bildung eines ersten Reaktorabstroms bestehend aus dem Beschickungskohlenwasserstoff, ungesättigtem Produktkohlenwasserstoff und Wasserstoff, einführt;

(b) den ersten Reaktorabstrom durch eine erste Leitung in einen zweiten Reaktor einführt und ihn mit Sauerstoff und dem Bett des selektiven Wasserstoffoxydationskatalysators in Berührung bringt, um den Abstrom durch die Oxydation des Sauerstoff zu erhitzen, den dabei erhitzten ersten Abstrom in Berührung mit einem zweiten Dehydrierungskatalysatorbett, das neben dem Bett des selektiven Wasserstoffoxydationskatalysator liegt, führt und einen zweiten Reaktorabstromom, bestehend aus Beschickungskohlenwasserstoff, ungesättigtem Produktkohlenwasserstoff und Wasserstoff, erzeugt;

(c) den zweiten Rekatorabstroom durch eine zweite Leitung in ein drittes Dehydrierungskatalysatorbett, welches größer ist als das zweite Dehydrierungskatalysatorbett, einführt und einen dritten Reaktorabstrom, bestehend aus dem Beschickungskohlenwasserstoff, ungesättigtem Produktkohlenwasserstoff un Wasserstoff, erzeugt; und

(d) den ungesättigten Produktkohlenwasserstoff gewinnt.

2. Verfahren nach Anspruch 1, dadurch gekennzeichnet, daß mit dem ersten Reaktorabstrom in der ersten Leitung Sauerstoff und Wasserdampf vermischt werden.

3. Verfahren nach Anspruch 1 oder 2, dadurch gekennzeichnet, daß der Beschickungskohlenwasserstoff ein alkylaromatischer Kohlenwasserstoff ist.

4. Verfahren nach einem der Ansprüche 1 bis 3, dadurch gekennzeichnet, daß das Bett des selektiven Wasserstoffoxydationskatalysators und das zweite Dehydrierungskatalysatorbett benachbarte Katalysatorringbetten mit einem gemeinsamen porösen Katalysatorauflagesieb sind.

5. Verfahren nach Anspruch 1, dadurch gekennzeichnet, daß der Beschickungskohlenwasserstoff Ethylbenzol ist, wobei der erste Reaktorabstrom aus Ethylbenzol, Wasserdampf, Styrol und Wasserstoff besteht, das Bett des selektiven Wasserstoffoxydationskatalysators gefahren wird, um den ersten Reaktorabstrom auf eine Temperatur oberhalb 605°C zu erhitzen, und der Abstrom des Bettes des selektiven Wasserstoffoxydationskatalysators in Berührung mit dem zweiten Dehydrierungskatalysatorbett geführt wird, wobei der zweite Reaktorabstrom eine Temperatur besitzt, die um wenigstens 10°C niedriger ist als der Abstrom des Bettes des selektiven Wasserstoffoxydationskatalysators.

**Revendications**

1. Procédé de déshydrogénation d'un hydrocarbure de départ, dans lequel on fait passer successivement l'hydrocarbure dans un premier lit de catalyseur de déshydrogénation pour former un mélange d'hydrocarbure de départ, d'un hydrocarbure insaturé produit et d'hydrogène; un lit de catalyseur d'oxydation sélective de l'hydrogène, en présence d'oxygène pour oxyder cet hydrogène; en un autre lit de catalyseur de déshydrogénation pour former encore un produit hydrocarboné insaturé; caractérisé en ce que ce procédé est mis en oeuvre dans au moins deux réacteurs séparés par des conduites; et en ce que:

(a) on fait passer l'hydrocarbure de départ dans un premier réacteur contenant le premier lit de catalyseur de déshydrogénation, pour former un premier courant de sortie de réacteur, comprenant l'hydrocarbure de départ, un produit hydrocarboné insaturé et de l'hydrogène;

(b) on transporte le premier courant de sortie de réacteur à travers une première conduite, jusqu'à un deuxième réacteur, et on le met en contact avec de l'oxygène et le lit de catalyseur d'oxydation sélective de l'hydrogène, pour chauffer le courant de sortie, par oxydation de l'hydrogène; on met le premier courant de sortie ainsi chauffé, en contact avec un deuxième lit de catalyseur de déshydrogénation, voisin du lit de catalyseur d'oxydation sélective de l'hydrogène; et on forme un deuxième courant de sortie de réacteur, comprenant l'hydrocarbure de départ, un produit hydrocarboné insaturé et de l'hydrogène;

(c) on transporte à travers une deuxième conduite, le deuxième courant de sortie de réacteur, jusqu'à un troisième lit de catalyseur de déshydrogénation, plus grand que le deuxième lit de catalyseur de déshydrogénation, et en forme un troisième courant de sortie de réacteur, comprenant l'hydrocarbure de départ, un produit hydrocarboné insaturé et de l'hydrogène; et

(d) on récupère l'hydrocarbure insaturé produit.

2. Procédé selon la revendication 1, caractérisé en ce que l'on mélange de l'oxygène et de la vapeur avec le premier courant de sortie de réacteur dans le première conduits.

3. Procédé selon la revendication 1 ou 2, caractérisé en ce que l'hydrocarbure de départ, est un hydrocarbure alkyl-aromatique.

4. Procédé selon l'une quelconque des revendications 1 à 3, caractérisé en ce que le lit de catalyseur d'oxydation sélective de l'hydrogène et le deuxième lit de catalyseur d'hydrogénation, sont des lits de catalyseur annulaires voisins, comprenant, en commun, un écran de retenue de catalyseur poreux.

5. Procédé selon la revendication 1, caractérisé en ce que l'hydrocarbure de départ est l'éthylbenzène, de sorte que le premier courant de sortie de réacteur, contient de de l'éthylbenzène, de la vapeur, du styrène et de l'hydrogène; on met en oeuvre le lit de catalyseur d'oxydation sélective de l'hydrogène, pour chauffer le premier courant de sortie de réacteur, à une température supérieure à 605°C; et on met l'effluent du lit de catalyseur d'oxydation sélective de l'hydrogène, en contact avec le deuxième lit de catalyseur de déshydrogénation, de sorte que le deuxième courant de sortie de réacteur a une température d'au moins 10°C en-dessous de celle de l'effluent du lit de catalyseur d'oxydation sélective de l'hydrogène.